# EUROPEAN PATENT APPLICATION

(11) **EP 2 364 733 A2**
(43) Date of publication of application: **14.09.2011**
(21) Application number: 11157780.5
(22) Date of filing: 11.03.2011
(51) Int. Cl.: A61K 47/02, A61K 47/10, A61K 47/22, A61K 9/70, A61K 31/137

(54) **Selegiline-Containing Adhesive Preparation**

(30) Priority: 12.03.2010 JP 2010056631
(71) Applicant: Nitto Denko Corporation, Ibaraki-shi, Osaka 567-8680 (JP); Fujimoto Co., Ltd., Matsubara-shi, Osaka (JP)
(72) Inventor: Inosaka, Keigo, Ibaraki-shi, Osaka 567-8680 (JP); Ameyama, Satoshi, Ibaraki-shi, Osaka 567-8680 (JP); Nakamura, Koji, Ibaraki-shi, Osaka 567-8680 (JP); Sekiya, Junichi, Ibaraki-shi, Osaka 567-8680 (JP)
(74) Representative: Grünecker, Kinkeldey, Stockmair & Schwanhäusser Anwaltssozietät

(57) **Abstract**

The present invention provides an adhesive preparation, which includes a backing and a pressure-sensitive adhesive layer formed on at least one side of the backing, the pressure-sensitive adhesive layer containing (-)-(R)-N,α-dimethyl-N-2-propynylphenethylamine and/or a pharmaceutically acceptable salt thereof, a pressure-sensitive adhesive and one or two or more stabilizers selected from the group consisting of 2-mercaptobenzimidazole, sodium sulfite, butylhydroxyanisole and butylhydroxytoluene.

## Description

### FIELD OF THE INVENTION

This invention relates to an adhesive preparation which comprises (-)-(R)-N,α-dimethyl-N-2-propynylphenethylamine (to be referred to as "free form of selegiline" hereinafter) and/or a pharmaceutically acceptable salt of (-)-(R)-N,α-dimethyl-N-2-propynylphenethylamine (to be referred to as "salt of selegiline" hereinafter, and both of this salt and the aforementioned "free form of selegiline" to be referred inclusively to as "selegiline").

### BACKGROUND OF THE INVENTION

An antiparkinsonism drug, selegiline, is known as an inhibitor of monoamine oxidase (MAO), and it is also known that there are different subtypes of MAO, i.e., type A (MAO-A) and type B (MAO-B), and selegiline is a selective inhibitor ofMAO-B. On the other hand, it is known that selegiline also inhibits MAO-A when it is orally administered in a large amount and shows anti-depression action. However, since a lot of MAO-A is present in the digestive organs, when MAO-A is inhibited by oral administration of selegiline, there is a possibility of causing sudden hypertension. Accordingly, an administration form of selegiline that has fewer possibility of transferring the drug to the digestive organs has been in demand.

In this regard, since an adhesive preparation can avoid absorption of a drug into the digestive organs and its first pass effect at the liver, it is considered that the adhesive preparation is suitable as an administration form in the case of administering selegiline in a large amount. However, there is a problem regarding the stability of selegiline in the preparation.

In addition, regarding a stabilizer for increasing stability of a percutaneous absorption drug in an adhesive preparation, several substances are known. For example, JP-A-11-79979 discloses that coloring of an adhesive preparation can be inhibited by containing 2-mereaptobenzimidazole and/or propyl gallate and a percutaneous absorption drug in a pressure-sensitive adhesive layer containing an acrylic copolymer. However, there is no disclosure or suggestion in this reference on selegiline and its stability.

### SUMMARY OF THE INVENTION

An object of the invention is to provide an adhesive preparation containing selegiline (to be referred to as "selegiline-containing adhesive preparation" hereinafter) in which the stability of selegiline is high and the amount of impurities formed is suppressed.

With the aim of attaining the above-mentioned object, the present inventors have conducted intensive studies on the stabilizing effects of various substances and found as a result that by allowing a specific stabilizer to be contained in a pressure-sensitive adhesive layer of an adhesive preparation that contains selegiline, there can be provided a selegiline-containing adhesive preparation which forms very small amount of selegiline-derived decomposition residues in the pressure-sensitive adhesive layer and is stable even after a long-term storage, thus resulting in the accomplishment of the invention.

Namely, the present invention provides the following items.
1. An adhesive preparation, which comprises a backing and a pressure-sensitive adhesive layer formed on at least one side of the backing, the pressure-sensitive adhesive layer comprising (-)-(R)-N,α-dimethyl-N-2-propynylphenethylamine and/or a pharmaceutically acceptable salt thereof, a pressure-sensitive adhesive and one or two or more stabilizers selected from the group consisting of 2-mercaptobenzimidazole, sodium sulfite, butylhydroxyanisole and butylhydroxytoluene.
2. The adhesive preparation according to item 1, wherein the stabilizer is 2-mercaptobenzimidazole.
3. The adhesive preparation according to item 1 or 2, wherein (-)-(R)-N,α-dimethyl-N-2-propynylphenethylamine and/or the pharmaceutically acceptable salt thereof is dissolved in the pressure-sensitive adhesive layer.
4. The adhesive preparation according to any one of items 1 to 3, wherein the pharmaceutically acceptable salt of (-)-(R)-N,α-dimethyl-N-2-propynylphenethylamine is a hydrochloride.
5. The adhesive preparation according to any one of items 1 to 4, wherein the pressure-sensitive adhesive layer further comprises a metal chloride.
6. The adhesive preparation according to any one of items 1 to 5, wherein the pressure-sensitive adhesive is an acrylic pressure-sensitive adhesive.
7. The adhesive preparation according to any one of items 1 to 6, wherein the pressure-sensitive adhesive layer is a crosslinked pressure-sensitive adhesive layer.
8. The adhesive preparation according to item 7, wherein the crosslinked pressure-sensitive adhesive layer has been crosslinked with a metal chelate compound.
9. The adhesive preparation according to any one of items 1 to 8, wherein the pressure-sensitive adhesive layer further comprises a liquid plasticizer.

According to the invention, there can be provided an adhesive preparation which is high in stability of selegiline and is low in the amount of impurities formed even after a long-term storage.

### DETAILED DESCRIPTION OF THE INVENTION

The following describes the invention in detail.

The adhesive preparation of the invention is for effecting percutaneous absorption of selegiline, contains selegiline in its pressure-sensitive adhesive layer and can be used as an antiparkinsonism drug and an antidepressant. In addition, as its other applications, there may be mentioned an anti-Alzheimer disease agent, an antiepileptic, seasickness prevention, treatment of schizophrenia, maintenance and protection of nerve cell function, improvement of acetylcholine system neurotransmitter, treatment of glaucoma, prevention of senescence, treatment of HIV-related cognition function disorder, treatment of ADHD (attention-deficit hyperactivity disorder) and the like.

Selegiline as the active ingredient of the adhesive preparation of the invention can be contained in the pressure-sensitive adhesive layer in a dissolved state, a dispersed state and/or a crystalline state.
When selegiline is contained in a crystalline state in the pressure-sensitive adhesive layer of the conventional adhesive preparations, its distribution into the pressure-sensitive adhesive layer varies depending on the crystalline shape and thus its discharge rate from the pressure-sensitive adhesive layer also varies, so that control of the crystalline shape becomes very important. However, a drug having polymorphism easily changes into a type of different crystal form due to outside factors (heat, impact, moisture, pressure and the like). Accordingly, there is a possibility that the control of the crystalline shape becomes difficult to attain.
When selegiline is contained in a dispersed state in the pressure-sensitive adhesive layer of the conventional adhesive preparations, dissolution of a portion of selegiline into the pressure-sensitive adhesive layer may be accelerated due to the heating in the drying step and the like during the production process, leading to crystallization therefrom. Accordingly, similar to the case of crystalline state, there is a possibility that control of the crystalline shape becomes necessary to carry out.
When selegiline is contained in a dissolved state in the pressure-sensitive adhesive layer of the conventional adhesive preparations, the aforementioned change in discharge rate does not occur. However, the dissolved state is relatively apt to undergo influences of environmental changes such as heat, a chemical reaction and the like, so that there is a possibility that impurities tend to form as a result. As is described later, forming amount of such impurities can be reduced in the adhesive preparation of the invention. Accordingly, the adhesive preparation of the invention is particularly advantageous in the case of an embodiment in which selegiline is contained in the pressure-sensitive adhesive layer in a dissolved state.

As the pharmaceutically acceptable salt of selegiline, for example, there may be mentioned an inorganic acid salt such as hydrochloride, hydrobromide, phosphate, nitrate, sulfate and the like and an organic acid salt such as acetate, oxalate, maleate, fumarate, tartrate, succinate and the like. Of these salts, hydrochloride (to be referred also to as "selegiline hydrochloride" hereinafter) is preferable from the viewpoint that when neutralized with a basic compound such as a metal hydroxide and the like, a metal chloride such as sodium chloride and the like, which inhibits reduction of cohesive strength and cohesive failure of the pressure-sensitive adhesive layer and thereby contributes to the stabilization of the preparation, can be formed.

Content of selegiline in the pressure-sensitive adhesive layer is within the range of from 0.5% by weight to 30% by weight, preferably from 1% by weight to 30% by weight, based on the total weight of the pressure-sensitive adhesive layer. When the content thereof is smaller than 0.5% by weight, there is a possibility that the desired therapeutic and preventive effects cannot be obtained, while when it is larger than 30% by weight, there is a possibility that a side effect due to high concentration selegiline is expressed.

As the backing to be used in the invention, although there is no particular limitation, a material in which contents of a liquid plasticizer and selegiline are not reduced due to their loss from the backside through the backing, namely a material having impermeability for these components, is desirable. Illustratively, there may be mentioned a film made of a polyester such as polyethylene terephthalate, nylon, polyvinyl chloride, polyethylene, polypropylene, an ethylene-vinyl acetate copolymer, polytetrafluoroethylene, an ionomer resin and the like, a metal foil or a laminate film thereof and the like. In addition, in order to improve adhesiveness (anchoring property) with the pressure-sensitive adhesive layer, it is possible to constitute the backing by a laminate film of a nonporous film made of the above-mentioned material with a porous film and form the pressure-sensitive adhesive layer on the porous film side.

The above-mentioned porous film is not particularly limited so long as the anchoring property of the pressure-sensitive adhesive layer is appropriate, and for example, there may be mentioned paper, woven fabric, non-woven fabric, a mechanically punching-treated sheet and the like, of which paper, woven fabric or non-woven fabric is particularly preferable. When improvement of the anchoring property and flexibility of the adhesive preparation are taken into consideration, thickness of such a porous film, is generally from about 10 µm to about 500 µm, and in the case of a thin adhesive preparation such as a plaster type or pressure-sensitive adhesive tape type, it is generally from about 10 µm to about 200 µm. In addition, in the case of woven fabric and non-woven fabric, it is desirable to set the weight of from 5 g/m² to 30 g/m² from the viewpoint of improving anchoring strength.

The pressure-sensitive adhesive layer according to the invention is formed on at least one side of the backing. As the pressure-sensitive adhesive to be contained in the pressure-sensitive adhesive layer of the invention, an acrylic pressure-sensitive adhesive, a rubber-based pressure-sensitive adhesive, a silicone-based pressure-sensitive adhesive, a vinyl ester-based pressure-sensitive adhesive and the like can be mentioned. Particularly, an acrylic pressure-sensitive adhesive containing an acrylic polymer is desirable from the viewpoint of skin adhesiveness as the adhesive preparation.

In general, the acrylic pressure-sensitive adhesive according to the invention is a polymer which comprises at least an alkyl ester of (meth)acrylic acid (to be also referred to as (meth)acrylic acid alkyl ester or alkyl (meth)acylate) as a monomer component, preferably a copolymer of an alkyl ester of (meth)acrylic acid with other monomer which is copolymerizable with the alkyl ester of (meth)acrylic acid (to be referred simply to as "other monomer" hereinafter), in which the main component is the alkyl ester of (meth)acrylic acid.

As the alkyl group of the (meth)acrylic acid alkyl ester, from the viewpoint of stickiness to the human skin, the number of carbon atoms is preferably 4 or more, particularly the number of carbon atoms is from 4 to 13, and it may be a straight chain or a branched chain. Illustratively, there may be mentioned butyl, isobutyl, sec-butyl, tert-butyl, pentyl, hexyl, heptyl, n-octyl, iso-octyl, sec-octyl, tert-octyl, 2-ethylhexyl, nonyl, decyl, undecyl, dodecyl, tridecyl and the like, of which 2-ethylhexyl of preferred. The (meth)acrylic acid alkyl ester can be used alone or by a combination of two or more species.

As the other monomer, examples thereof include carboxyl group-containing monomers such as (meth)acrylic acid, itaconic acid, maleic acid, maleic anhydride and the like; sulfoxyl group-containing monomers such as styrene sulfonate, allyl sulfonate, sulfopropyl (meth)acrylate, (meth)acryloyloxynaphthalene sulfonate, acrylamidomethyl sulfonate and the like; hydroxyl group-containing monomers such as hydroxyethyl (meth)acrylate, hydroxypropyl(meth)acrylate; (meth)acrylic acid derivatives having amido group such as (meth)acrylamide, dimethyl (meth)acrylamide, hydroxyethyl (meth)acrylamide, N-butyl (meth)acrylamide, N-methylol (meth)acrylamide and the like; aminoalkyl esters of (meth)acrylic acid such as aminoethyl (meth)acrylate, dimethylaminoethyl (meth)acrylate, tert-butylaminoethyl (meth)acrylate and the like; alkoxy esters of (meth)acrylic acid such as methoxyethyl (meth)acrylate, ethoxyethyl (meth)acrylate, tetrahydrofurfuryl (meth)acrylate and the like; alkoxyalkylene glycol esters of (meth)acrylic acid such as methoxyethylene glycol (meth)acrylate, methoxydiethylene glycol (meth)acrylate, methoxypolyethylene glycol (meth)acrylate, methoxypolypropylene glycol (meth)acrylate and the like; (meth)acrylonitrile; compounds having vinyl group such as vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole, vinylmorpholine and the like, and these may be used alone or as a combination of two or more species. Particularly, carboxyl group-containing monomers (preferably acrylic acid), hydroxyl group-containing monomers (preferably 2-hydroxyethyl acrylate), (meth)acrylic acid derivatives having amido group (preferably hydroxyethyl (meth)acrylamide), N-vinyl-2-pyrrolidone, vinyl acetate and the like are desirable from the viewpoint of pressure-sensitive adhesive characteristics.

Copolymerization ratio of the alkyl ester of (meth)acrylic acid and other monomer is not particularly limited and is arbitrarily set in response to the molecular weight characteristics of the copolymer to be obtained, such as weight average molecular weight and the like. Particularly preferable is a copolymer obtained by blending the alkyl ester of (meth)acrylic acid and other monomer at a weight ratio of alkyl ester of (meth)acrylic acid/other monomer = generally 50 to 97/50 to 3, preferably 65 to 95/35 to 5, followed by copolymerization.

As a desirable copolymer, for example, there may be mentioned a copolymer of 2-ethylhexyl acrylate, N-vinyl-2-pyrrolidone and acrylic acid; a copolymer of 2-ethylhexyl acrylate, 2-hydroxyethyl acrylate and vinyl acetate; a copolymer of 2-ethylhexyl acrylate and acrylic acid, and the like. From the viewpoint of pressure-sensitive adhesive characteristics of the copolymer, more preferred is a copolymer of 2-ethylhexyl acrylate, N-vinyl-2-pyrrolidone and acrylic acid, and particularly preferred is a copolymer obtained by blending 2-ethylhexyl acrylate, N-vinyl-2-pyrrolidone and acrylic acid at a weight ratio of 2-ethylhexyl acrylate/N-vinyl-2-pyrrolidone/acrylic acid = 50 to 90/10 to 30/0 to 5, followed by copolymerization.

Content of the pressure-sensitive adhesive in the pressure-sensitive adhesive layer is within the range of from 20% by weight to 90% by weight, preferably from 30% by weight to 90% by weight, based on the total weight of the pressure-sensitive adhesive layer. When the content thereof is smaller than 20% by weight, there is a possibility that it becomes difficult to maintain the skin adhesive strength of the adhesive preparation.

The adhesive preparation of the invention contains a stabilizer in its pressure-sensitive adhesive layer. By containing a stabilizer, amount of impurities formed in the pressure-sensitive adhesive layer can be reduced effectively. As such a stabilizer, for example, there may be mentioned at least one or more substances selected from the group consisting of 2-mercaptobenzimidazole, sodium sulfite, butylhydroxyanisole and butylhydroxytoluene. Of which, 2-mercaptobenimidazole is desirable because generation amount of impurities can be effectively reduced.

Content of the stabilizer in the pressure-sensitive adhesive layer is within the range of generally from 0.01 % by weight to 5.0% by weight, preferably from 0.02% by weight to 2.0% by weight, based on the total weight of the pressure-sensitive adhesive layer. When the content thereof is smaller than 0.01% by weight, the effect of the stabilizer may not be expressed, while when it is larger than 5.0% by weight, skin irritation due to the stabilizer may occur.

A liquid plasticizer may be contained in the pressure-sensitive adhesive layer of the adhesive preparation of the invention. When a liquid plasticizer is contained in the pressure-sensitive adhesive layer, the pressure-sensitive adhesive layer is softened and thus skin irritation during wearing and/or at the time of peeling can be reduced. As such a liquid plasticizer, there is no particular limitation so long as the substance itself is liquid at 25°C, shows plasticizing action and is compatible with an adhesive polymer constituting the above-mentioned pressure-sensitive adhesive, and the substance which can improve percutaneous absorption property and storage stability of selegiline is desirable. In addition, a liquid plasticizer can also be blended for the purpose of further increasing solubility and the like of selegiline in the pressure-sensitive adhesive.

As such a liquid plasticizer, there may be mentioned a fatty acid ester containing a higher fatty acid having from 12 to 16 carbon atoms and a lower monovalent alcohol having from 1 to 4 carbon atoms (to be referred also to as "C12-16/C1-4 fatty acid ester" hereinafter); a fatty acid having 8 or 9 carbon atoms, such as caprylic acid (octanoic acid, C8), pelargonic acid (nonanoic acid, C9) and the like; a glycerol ester of middle chain fatty acid; glycols such as ethylene glycol, diethylene glycol, triethylene glycol, polyethylene glycol, propylene glycol, 1,3-propanediol, polypropylene glycol and the like; oils and fats such as olive oil, castor oil, squalene, lanolin and the like; an organic solvent such as ethyl acetate, ethyl alcohol, dimethyl sulfoxide, dimethylformamide, dimethylacetamide, dimethyllaurylamide, dodecylpyrrolidone, isosorbitol, oleyl alcohol, lauric acid, oleic acid, N-methyl-2-pyrrolidone and the like; a liquid surfactant; hydrocarbons such as liquid paraffin; a conventionally known plasticizer such as phthalic acid ester and the like; as well as ethoxylated stearyl alcohol, isotridecyl myristate, ethyl oleate, adipic acid diester, sebacic acid diester, octyl palmitate, glycerol and the like. These liquid plasticizers may be used as one species alone or by a combination of two or more species.

In the above-mentioned C12-16/C1-4 fatty acid ester, the higher fatty acid having from 12 to 16 carbon atoms includes saturated and unsaturated fatty acids but a saturated fatty acid is desirable, and the lower monovalent alcohol having from 1 to 4 carbon atoms may be a straight chain or a branched chain. As the suitable examples of the higher fatty acid having from 12 to 16 carbon atoms, lauric acid (C12), myristic acid (C14) and palmitic acid (C16) may be mentioned, and as the suitable examples of the lower monovalent alcohol having from 1 to 4 carbon atoms, isopropyl alcohol, ethyl alcohol, methyl alcohol, propyl alcohol and the like may be mentioned. As the suitable illustrative examples of the fatty acid ester, isopropyl myristate, ethyl laurate and isopropyl palmitate may be mentioned.

As the glycerol ester of middle chain fatty acid (middle chain fatty acid ester of glycerol), a glycerol ester of a fatty acid having from 8 to 12 carbon atoms is desirable, and it may be any one of monoglyceride, diglyceride and triglyceride. The fatty acid having from 8 to 12 carbon atoms includes saturated and unsaturated fatty acids but a saturated fatty acid is desirable, and for example, there may be mentioned caprylic acid (octanoic acid, C8), pelargonic acid (nonanoic acid, C9), capric acid (decanoic acid, C10) and the like. As the particularly desirable glycerol esters of middle chain fatty acid, a middle chain fatty acid diglyceride, a middle chain fatty acid triglyceride and the like may be mentioned, of which a middle chain fatty acid triglyceride is most desirable.

As the middle chain fatty acid triglyceride, preferred is a triglyceride in which at least one of the three fatty acids bonding to glycerol by an ester bond is a middle chain fatty acid (the number of carbons therein is from 8 to 12), more preferred is a triglyceride in which at least two of the three fatty acids bonding to glycerol by an ester bond are a middle chain fatty acid (the number of carbons therein is from 8 to 12), and most preferred is a triglyceride in which all of the three fatty acids bonding to glycerol by an ester bond are a middle chain fatty acid (the number of carbons therein is from 8 to 12).

Also, in the middle chain fatty acid triglyceride, a triglyceride in which the middle chain fatty acid species (in which the number of carbons is from 8 to 12) that bonds to glycerol by an ester bond is only one species (e.g., caprylic acid triglyceride in which the middle chain fatty acid bonding to glycerol by an ester bond is (caprylic acid alone, capric acid triglyceride in which the middle chain fatty acid bonding to glycerol by an ester bond is capric acid alone, and the like) may be used, or a triglyceride in which the middle chain fatty acid species (in which the number of carbons is from 8 to 12) that bonds to glycerol by an ester bond are two or more species (e.g., (caprylic acid/capric acid) triglyceride in which the middle chain fatty acids that bond to glycerol by an ester bond are caprylic acid and capric acid, (caprylic acid/capric acid/lauric acid) triglyceride in which the middle chain fatty acids that bond to glycerol by an ester bond are caprylic acid, capric acid and lauric acid, and the like) may be used. As the middle chain fatty acid triglyceride in the invention, one species of middle chain fatty acid triglyceride alone may be used or a mixture of two or more species of middle chain fatty acid triglyceride may be used.

In addition, the middle chain fatty acid triglyceride may be an extract from a natural material or a synthesized product. In addition, a commercial item can also be used, and for example, there may be mentioned "COCONARD" manufactured by Kao Corp., "Crodamol GTCC" manufactured by Croda Inc., "PANACET 810S" manufactured by NOF CORPORATION and the like.

As the middle chain fatty acid diglyceride (in which the number of carbons is from 8 to 12), for example, caprylic acid diglyceride in which the middle chain fatty acid is caprylic acid alone may be mentioned. The middle chain fatty acid diglyceride may be an extract from a natural material or a synthesized product. In addition, a commercial item can also be used.

Preferred as the adipic acid diester is a diester in which the number of carbons of the alcohol residue that bonds to adipic acid by a ester bond is from 1 to 5, and for example, there may be mentioned dimethyl adipate, diethyl adipate, diisopropyl adipate, dibutyl adipate and the like, of which diisopropyl adipate is particularly desirable.

Preferred as the sebacic acid diester is a diester in which the number of carbons of the alcohol residue that bonds to sebacic acid by a ester bond is from 1 to 4, and for example, there may be mentioned dimethyl sebacate, diethyl sebacate, diisopropyl sebacate and the like, of which diisopropyl sebacate is particularly desirable.

According to the invention, from the viewpoint of compatibility with a pressure-sensitive adhesive (particularly an acrylic pressure-sensitive adhesive), storage stability of selegiline and the like, the liquid plasticizer is preferably a C12-16/C1-4 fatty acid ester, a fatty acid having 8 or 9 carbon atoms, a middle chain fatty acid glycerol ester or an adipic acid diester, more preferably a C12-16/C1-4 fatty acid ester, a middle chain fatty acid glycerol ester or an adipic acid diester, particularly preferably isopropyl myristate, a middle chain fatty acid triglyceride (e.g., (caprylic acid/capric acid) triglyceride) or diisopropyl adipate.

Content of the liquid plasticizer when the pressure-sensitive adhesive layer contains the liquid plasticizer is within the range of, for example, from 2% by weight to 60% by weight, preferably from 20% by weight to 50% by weight, more preferably from 30% by weight to 50% by weight, based on the total weight of the pressure-sensitive adhesive layer. When the content thereof is less than 2% by weight, there may be a case in which the skin irritation cannot be reduced due to insufficient plasticization of the pressure-sensitive adhesive layer. When it exceeds 60% by weight on the contrary, there may be a case in which the liquid plasticizer cannot be kept in the pressure-sensitive adhesive even by the cohesive force possessed by the pressure-sensitive adhesive, and there may be a case in which the adhesiveness becomes poor due to the blooming of the liquid plasticizer on the surface of the pressure-sensitive adhesive layer. In addition, by crosslinking a pressure-sensitive adhesive layer containing 20% by weight or more of the liquid plasticizer, it becomes possible to provide an adhesive preparation which has softness and shows low skin irritation at the time of peeling.

In addition, in the adhesive preparation of the invention, a crosslinking agent may be contained in the pressure-sensitive adhesive layer in order to apply a crosslinking treatment to the pressure-sensitive adhesive layer. As such a crosslinking agent, for example, an organic metal compound, a metal alcoholate, a metal chelate compound and the like may be mentioned. Illustratively, examples of the organic metal compound include zirconium, zinc alaninate, zinc acetate, glycine ammonium zinc and the like. Examples of the metal alcoholate include tetraethyl titanate, tetraisopropyl titanate, aluminum isopropylate, aluminum sec-butylate and the like. Examples of the metal chelate compound include di-iso-propoxybis(acetylacetone) titanate, tetraoctylene glycol titanate, aluminum isopropylate, ethyl acetoacetate aluminum diisopropylate, aluminum tris(ethyl acetoacetate), aluminum tris(acetyl acetonate) and the like. In this connection, a suitably used crosslinking agent is a metal chelate compound. Particularly, ethyl acetoacetate aluminum diisopropylate is more preferable. For the crosslinking treatment, the above-mentioned crosslinking agents may be used alone or as a combination of two or more species.

Blending amount of the crosslinking agent in the case of applying a crosslinking treatment to the pressure-sensitive adhesive layer varies depending on the kinds of the crosslinking agent and the pressure-sensitive adhesive, but is generally from 0.05% by weight to 0.6% by eight based on the total weight of the pressure-sensitive adhesive layer.

In addition, from the viewpoint of inhibiting influence of lactic acid in perspiration, a metal chloride may be contained in the pressure-sensitive adhesive layer of the adhesive preparation of the invention. As such a metal chloride, sodium chloride, calcium chloride, aluminum chloride, stannous chloride, ferric chloride and the like metal chlorides may be generally mentioned from the viewpoint of safety. Particularly, preferred is sodium chloride and calcium chloride, and more preferred is sodium chloride. Any one of these may be used alone, or two or more thereof may be used in combination.

The metal chloride according to the invention may be a salt formed by neutralizing selegiline hydrochloride with a basic compound. Illustratively, in the case of using selegiline hydrochloride as a salt of selegiline, a metal chloride formed by neutralizing selegiline hydrochloride while mixing and stirring together with a basic compound in a solvent comes under this. Owing to this neutralization, a drug-containing liquid which contains a metal chloride can be prepared without adding metal chloride. In addition, after forming the metal chloride by mixing and stirring selegiline hydrochloride together with a basic compound in a solvent, the metal chloride may further be added to the thus obtained drug-containing liquid. As such a basic compound, a metal hydroxide is desirable, and as the metal hydroxide, for example, there may be mentioned sodium hydroxide, aluminum hydroxide, potassium hydroxide, calcium hydroxide, magnesium hydroxide an the like, of which sodium hydroxide is desirable.

Blending amount of the metal chloride is generally from 0.1 part by weight to 20 parts by weight, preferably from 1 part by weight to 10 parts by weight, based on 100 parts by weight of the pressure-sensitive adhesive polymer constituting the above-mentioned pressure-sensitive adhesive. When this blending amount is less than 0.1 part by weight, there may be a case in which the effect of inhibiting the influence by lactic acid in perspiration becomes insufficient, while when it exceeds 20 parts by weight, there may be a case in which it causes a poor appearance due to its inability to disperse uniformly in the pressure-sensitive adhesive polymer, though there is an inhibitory effect.

From the viewpoint of attaching onto the skin surface and peeling therefrom, thickness of the pressure-sensitive adhesive layer is generally from 10 µm to 300 µm, preferably from 50 µm to 200 µm.

According to the necessity, the pressure-sensitive adhesive layer can be blended with additive agents such as an antioxidant, various pigments, various fillers, a drug solubilizing agents, a drug dissolution inhibitor and the like.

From the viewpoint of adhesion to skin, the pressure-sensitive adhesive layer is preferably a hydrophobic pressure-sensitive adhesive layer and more preferably a non-hydroscopic pressure-sensitive adhesive layer. The term "non-hydroscopic pressure-sensitive adhesive layer" as used herein is not always limited to those which are completely free from, moisture, but those which contain a slight amount of moisture derived from the air humidity, the skin and the like are included therein. The term "a slight amount of moisture" as used herein is, as the moisture content of the layered product of backing and pressure-sensitive adhesive layer, preferably 5% by weight or less, more preferably 2% by weight or less, most preferably 1% by weight or less. In this case, the moisture content of the layered product of backing and pressure-sensitive adhesive layer means weight ratio of water contained in the layered product of backing and pressure-sensitive adhesive layer after separating a release liner when present (i.e., weight percentage of water based on the total weight of the layered product of backing and pressure-sensitive adhesive layer) which is measured by the coulometric Karl Fischer titration method, and is illustratively as follows. That is, under an environment controlled at a temperature of 23 ± 2°C and a relative humidity of 40 ± 5% RH, a test piece is prepared by punching a sample having a release liner when present, into a predetermined size. Then, after peeling off the release liner when present, the resulting test piece is put into a moisture vaporizer. The test piece is heated at 140°C in the moisture vaporizer, the moisture generated is then introduced into a titration flask using nitrogen as the carrier, and the moisture content (% by weight) of the sample is measured by the coulometric Karl Fischer titration method.

The production method of the adhesive preparation of the invention is not particularly limited, but for example, it can be produced by the following production method.

Firstly, a drug-containing solution is prepared by mixing and stirring free form of selegiline and/or a salt of selegiline with a metal chloride dispersed in a solvent such as ethanol. In this case, when selegiline hydrochloride is used as the salt of selegiline, the metal chloride may be formed by neutralizing selegiline hydrochloride by mixing and stirring it together with the above-mentioned metal hydroxide and the like in a solvent. Also, after forming the metal chloride by mixing and stirring selegiline hydrochloride together with the metal hydroxide in a solvent, the metal chloride may further be added to the thus obtained drug-containing liquid.

The above-mentioned drug-containing solution is, for example, dissolved or dispersed in a solvent or dispersion medium together with a pressure-sensitive adhesive (e.g., an acrylic copolymer pressure-sensitive adhesive and the like), a stabilizer, and, in response to the necessity, a crosslinking agent, a liquid plasticizer, other additives and the like. In this connection, since the salt of selegiline has low solubility for the pressure-sensitive adhesive layer, there is a tendency to form a dispersed state. The solvent or dispersion medium to be used in forming the pressure-sensitive adhesive layer is not particularly limited, and those which are generally used as a solvent and the like for a pressure-sensitive adhesive can be selected by taking kind of the pressure-sensitive adhesive, its reactivity with the drug, and the like into consideration. As such a solvent or dispersion medium, ethyl acetate, toluene, hexane, 2-propanol, methanol, ethanol and the like may for example be mentioned.

Next, a pressure-sensitive adhesive layer is formed by coating the thus obtained solution or dispersion on one side of the backing or the release treatment side of a release sheet, followed by drying. In this connection, it is possible to carry out the aforementioned coating by, for example, a technique conventionally known to those skilled in the art, such as casting, printing and the like. Thereafter, the release sheet or backing is pasted to the pressure-sensitive adhesive layer. As such a release sheet, there is no particular limitation so long as it can be easily peeled off from the pressure-sensitive adhesive layer when used, and for example, there may be used a film such as of polyester, polyvinyl chloride, polyvinylidene chloride, polyethylene terephthalate and the like in which a silicone treatment was applied to its contacting side with the pressure-sensitive adhesive layer, or a laminated film of wood free paper or glassine paper with polyolefin, and the like. Thickness of the release sheet is generally 200 µm or less, preferably from 25 µm to 100 µm. In this connection, when a crosslinking treatment is carried out, the adhesive preparation of the invention is prepared by, after pasting the release sheet to the pressure-sensitive adhesive layer, accelerating the crosslinking reaction by applying an aging treatment and the like at generally from 60°C to 90°C, preferably from 60°C to 70°C, for a period of from 24 hours to 48 hours.

In this connection, the adhesive preparation may also be formed as follows. Namely, after preparing a drug-containing solution by dissolving or dispersing selegiline in a solvent or dispersion medium together with a pressure-sensitive adhesive (e.g., an acrylic copolymer pressure-sensitive adhesive and the like), a stabilizer, and, in response to the necessity, a liquid plasticizer, a crosslinking agent, other additives and the like, the thus obtained solution is subsequently mixed with a basic compound and/or a metal chloride while stirring. Thereafter, the solution is then coated on one side of the backing or the release treatment side of a release sheet, followed by drying, thereby forming a pressure-sensitive adhesive layer. Then, the pressure-sensitive adhesive layer is pasted to the release sheet or backing.

Shape of the adhesive preparation of the invention is not limited, and for example, it may be a tape shape, a sheet shape and the like.

Dose of the adhesive preparation of the invention varies depending on the age, body weight, symptoms and the like of each patient, but it is desirable to apply an adhesive preparation containing from 1 mg to 40 mg of selegiline, generally to the skin of an adult within an area of from 1 cm² to 40 cm² approximately from once per two days to twice a day.

### Examples

The following describes the invention in detail with reference to examples, though the invention is not limited to these examples. In this connection, the term "part(s)" as used in the following descriptions means "part(s) by weight".

### (Preparation of acrylic pressure-sensitive adhesive)

Under an inert gas atmosphere, 72 parts of 2-ethylhexyl acrylate, 25 parts of N-vinyl-2-pyrrolidone, 3 parts of acrylic acid and 0.2 part of azobisisobutyronitrile were allowed to undergo solution polymerization in ethyl acetate at 60°C, thereby preparing a solution of an acrylic pressure-sensitive adhesive. The weight average molecular weight of the acrylic pressure-sensitive adhesive was about 1,800,000.

### (preparation of selegiline-containing adhesive preparations of Examples 1 to 9 and Comparative Examples 1 to 5)

Each pressure-sensitive adhesive solution was prepared in accordance with the blending ratio shown in the following Table 1, and the thus obtained solution was coated on a polyester film (75 µm in thickness) so that the thickness of a pressure-sensitive adhesive layer after drying became 80 µm and then dried to prepare the pressure-sensitive adhesive layer. Subsequently, this pressure-sensitive adhesive layer was pasted on a polyester film (12 µm in thickness) and then an aging treatment was carried out at 60°C for 48 hours, thereby preparing a selegiline-containing adhesive preparation. In this connection, in the table, IPM represents isopropyl myristate, and ALCH represents ethyl acetoacetate aluminum diisopropylate, and GTCC represents a middle chain fatty acid triglyceride ((caprylic acid/capric acid) triglyceride, "COCONARD MT" mfd. by Kao Corp.). In addition, all the units in the table are "% by weight" based on the total weight of the pressure-sensitive adhesive layer.

**Table 1**

| | Pressure-sensitive adhesive | Selegiline hydrochloride | Stabilizer | | Sodium hydroxide | Plasticizer | | ALCH |
|---|---|---|---|---|---|---|---|---|
| | | | Name | Content | | Name | Content | |
| Example 1 | 31.00 | 24.00 | 2-Mercaptobenzimidazole | 0.62 | 4.29 | IPM | 40.00 | 0.09 |
| Example 2 | 31.12 | 24.00 | Sodium sulfite | 0.50 | 4.29 | IPM | 40.00 | 0.09 |
| Example 3 | 31.12 | 24.00 | Butylhydroxyanisole | 0.50 | 4.29 | IPM | 40.00 | 0.09 |
| Example 4 | 31.12 | 24.00 | Butylhydroxytoluene | 0.50 | 4.29 | IPM | 40.00 | 0.09 |
| Example 5 | 45.77 | 11.70 | 2-Mercaptobenzimidazole | 0.30 | 2.09 | IPM | 40.00 | 0.14 |
| Example 6 | 50.76 | 11.70 | 2-Mercaptobenzimidazole | 0.30 | 2.09 | GTCC | 35.00 | 0.15 |
| Example 7 | 87.09 | 9.00 | 2-Mercaptobenzimidazole | 0.30 | 1.61 | IPM | 2.00 | - |
| Example 8 | 87.29 | 9.00 | 2-Mercaptobenzimidazole | 0.10 | 1.61 | IPM | 2.00 | - |
| Example 9 | 85.55 | 12.00 | 2-Mercaptobenzimidazole | 0.30 | 2.15 | - | - | - |
| Comparative Example 1 | 31.60 | 24.00 | L(+)-Ascorbic acid | 0.05 | 4.29 | IPM | 40.00 | 0.09 |
| Comparative Example 2 | 31.12 | 24.00 | L-Ascorbyl palmitate | 0.02 | 4.29 | IPM | 40.00 | 0.09 |
| Comparative Example 3 | 31.12 | 24.00 | 3-Mercapto-1,2-propanediol | 0.50 | 4.29 | IPM | 40.00 | 0.09 |
| Comparative Example 4 | 31.12 | 24.00 | Sodium metabisulfite | 0.50 | 4.29 | IPM | 40.00 | 0.09 |
| Comparative Example 5 | 31.61 | 24.00 | - | - | 4.29 | IPM | 40.00 | 0.10 |

### (Evaluation tests)

Each of the adhesive preparations of Examples 1 to 4 and Comparative Examples 1 to 5 was preserved for 1 month under respective temperature conditions of 40°C, 50°C and 60°C, and then evaluation tests were carried out on the stability and preparation coloring degree of these adhesive preparations.
In addition, each of the adhesive preparations of Examples 1 and 5 to 9 was preserved at 50°C for 3 months or at 40°C for 6 months, and then evaluation test was carried out on the stability of these adhesive preparations.

### (Stability)

Evaluation method of the stability is as follows.
Each adhesive preparation was punched into an appropriate size and extracted with an organic solvent on a shaker, and the content of impurities in the extracted solution was measured using high performance liquid chromatography (HPLC). Illustratively, peak areas of selegiline hydrochloride and impurities (relative retention time based on selegiline: 0.78) were respectively measured, and the ratio of peak area of the impurities to peak area of selegiline was multiplied by 100 and used as the content of impurities (%).

The measuring conditions of HPLC are as follows.
Detector: An ultraviolet ray absorption photometer (wavelength; 205 nm)
Column: Kaseisorb LC ODS 2000 (mfd. by Tokyo Chemical Industry)
Column temperature: 25°C
Flow rate: 0.9 ml/min
A mobile phase liquid A and a mobile phase liquid B were mixed at the ratio described in the following Table 2 and used as the mobile phase. In this connection, preparation of the mobile phase liquid A and mobile phase liquid B was carried out as described in the followings.
Mobile phase liquid A: A 11.50 g portion of ammonium dihydrogenphosphate was dissolved in 1000 ml of water and adjusted to pH 3.1 with phosphoric acid. A 100 ml portion of acetonitrile for liquid chromatography was added to 900 ml of this solution and mixed to prepare the mobile phase liquid A.
Mobile phase liquid B: A 11.50 g portion of ammonium dihydrogenphosphate was dissolved in 1000 ml of water and adjusted to pH 3.1 with phosphoric acid. A 350 ml portion of acetonitrile for liquid chromatography was added to 650 ml of this solution and mixed to prepare the mobile phase liquid B.

**Table 2**

| Time (min) | Mobile phase liquid A/mobile phase liquid B (volume/volume ratio) |
|---|---|
| 0 | 100/0 |
| 5 | 90/10 |
| 25 | 0/100 |
| 35 | 0/100 |
| 35.01 | 100/0 |
| 40 | 100/0 |

### (Preparation coloring degree)

Evaluation method of the preparation coloring degree is as follows.
Each adhesive preparation was punched into an appropriate size and used as a measuring sample, and using Chroma Meter CR-200 (mfd. by MINOLTA CAMERA CO., LID.), the value of b* was measured by the color measure (L*a*b*) mode. The white calibration plate attached thereto was used for the calibration of the apparatus, and measurement was carried out by putting the measuring sample on said white calibration plate. In this connection, the "L*a*b*" according to the invention means the L*a*b* color system standardized in 1976 by CIE (Commission Internationale de l'Eclairage) (CIE 1976) [in accordance with JIS Z 8729 (1980)]. The L* represents psychometric lightness, and a* and b* represent psychometric chroma coordinates. Illustratively, regarding the value of b*, (+) represents a change to yellow, and (-) a change to blue.

**Table 3**

| | Initial amount of impurities (%) | Storage condition | Amount of impurities after storage (%) | Value of b* |
|---|---|---|---|---|
| Example 1 | 0.03 (initial b* value = 3.06) | 60°C | 0.09 | 3.95 |
| | | 50°C | 0.04 | 3.27 |
| | | 40°C | 0.04 | 3.10 |
| Example 2 | N.D. (initial b* value = 3.01) | 60°C | 0.23 | 3.89 |
| | | 50°C | N. D. | 3.41 |
| | | 40°C | N.D. | 3.16 |
| Example 3 | 0.06 | 60°C | 0.33 | 5.67 |
| | | 50°C | 0.13 | 4.92 |
| | | 40°C | 0.09 | 4.43 |
| Example 4 | 0.08 | 60°C | 0.43 | 5.41 |
| | | 50°C | 0.13 | 4.42 |
| | | 40°C | 0.08 | 4.86 |
| Comparative Example 1 | 0.08 | 60°C | 0.63 | 7.00 |
| | | 50°C | 0.24 | 5.10 |
| | | 40°C | 0.15 | 4.04 |
| Comparative Example 2 | 0.08 | 60°C | 0.68 | 6.61 |
| | | 50°C | 0.23 | 4.82 |
| | | 40°C | 0.16 | 4.43 |
| Comparative Example 3 | 0.10 | 60°C | 0.77 | 7.64 |
| | | 50°C | 0.50 | 5.01 |
| | | 40°C | 0.34 | 4.61 |
| Comparative Example 4 | 0.07 | 60°C | 0.44 | 4.86 |
| | | 50°C | 0.46 | 3.62 |
| | | 40°C | 0.30 | 3.40 |
| Comparative Example 5 | 0.08 (initial b* value = 3.08) | 60°C | 0.30 | 9.64 |
| | | 50°C | 0.24 | 3.82 |
| | | 40°C | 0.12 | 3.37 |

**Table 4**

| | Initial amount of impurities (%) | Storage condition | Amount of impurities after storage (%) |
|---|---|---|---|
| Example 1 | 0.03 | 50°C, 3 months | 0.09 |
| | | 40°C, 6 months | 0.07 |
| Example 5 | 0.01 | 50°C, 3 months | 0.06 |
| | | 40°C, 6 months | 0.06 |
| Example 6 | 0.02 | 50°C, 3 months | 0.08 |
| | | 40°C, 6 months | 0.08 |
| Example 7 | 0.02 | 50°C, 3 months | 0.12 |
| | | 40°C, 6 months | 0.10 |
| Example 8 | 0.02 | 50°C, 3 months | 0.13 |
| | | 40°C, 6 months | 0.10 |
| Example 9 | 0.01 | 50°C, 3 months | 0.12 |
| | | 40°C, 6 months | 0.12 |

As is evident from Table 3, amounts of impurities formed was reduced in Examples 1 to 4 which used the specific stabilizers of the invention, in comparison with Comparative Example 5 to which the stabilizer was not added, In addition, in Examples 1 and 2, formation of impurities was inhibited even under the severe storage condition of 60°C. Particularly, the stability of Example 1 under high temperature condition was worthy of special mention. Contrary to this, in Comparative Examples 1 to 4 in which not the specific stabilizers of the invention but other stabilizers were used, formation of impurities was not inhibited, and far from this, was rather increased than or the same level the case of Comparative Example 5 to which nothing was added. Regarding the value of b*, Examples 1 and 2 and were good and Example 1 was particularly good in comparison with Comparative Example 5 in which none of the stabilizers was added.
In addition, as is evident from Table 4, formation of impurities was inhibited in Examples 1 and 5 to 9, even after storage at 50°C for 3 months or at 40°C for 6 months.

While the present invention has been described in detail and with reference to specific embodiments thereof, it will be apparent to one skilled in the art that various changes and modifications can be made therein without departing from the scope thereof.

This application is based on Japanese patent application No. 2010-056631 filed March 12, 2010, the entire contents thereof being hereby incorporated by reference.

## Claims

1. An adhesive preparation, which comprises a backing and a pressure-sensitive adhesive layer formed on at least one side of the backing, the pressure-sensitive adhesive layer comprising (-)-(R)-N,α-dimethyl-N-2-propynylphenethylamine and/or a pharmaceutically acceptable salt thereof, a pressure-sensitive adhesive and one or two or more stabilizers selected from the group consisting of 2-mercaptobenzimidazole, sodium sulfite, butylhydroxyanisole and butylhydroxytoluene.

2. The adhesive preparation according to claim 1, wherein the stabilizer is 2-mercaptobenzimidazole.

3. The adhesive preparation according to claim 1 or 2, wherein (-)-(R)-N,α-dimethyl-N-2-propynylphenethylamine and/or the pharmaceutically acceptable salt thereof is dissolved in the pressure-sensitive adhesive layer.

4. The adhesive preparation according to any one of claims 1 to 3, wherein the pharmaceutically acceptable salt of (-)-(R)-N,α-dimethyl-N-2-propynylphenethylamine is a hydrochloride.

5. The adhesive preparation according to any one of claims 1 to 4, wherein the pressure-sensitive adhesive layer further comprises a metal chloride.

6. The adhesive preparation according to any one of claims 1 to 5, wherein the pressure-sensitive adhesive is an acrylic pressure-sensitive adhesive.

7. The adhesive preparation according to any one of claims 1 to 6, wherein the pressure-sensitive adhesive layer is a crosslinked pressure-sensitive adhesive layer.

8. The adhesive preparation according to claim 7, wherein the crosslinked pressure-sensitive adhesive layer has been crosslinked with a metal chelate compound.

9. The adhesive preparation according to any one of claims 1 to 8, wherein the pressure-sensitive adhesive layer further comprises a liquid plasticizer.
